# EUROPÄISCHE PATENTSCHRIFT

(11) **EP 0 626 375 B1**
(45) Veröffentlichungstag und Bekanntmachung des Hinweises auf die Patenterteilung: **05.06.2002**
(21) Anmeldenummer: 94107543.4
(22) Anmeldetag: 16.05.1994
(51) Int. Cl.: C07D 273/00, A61K 31/395, C07K 11/02, C07K 11/00

(54) **Octacylodepsideptide mit endoparasitizider Wirkung**
Octacyclodepsipeptides having endoparasiticidal activity
Octacyclodepsipeptides ayant une activité endoparasiticide

(30) Priorität: 26.05.1993 DE 4317457
(43) Veröffentlichungstag der Anmeldung: 30.11.1994
(73) Patentinhaber: BAYER AG, 51368 Leverkusen (DE)
(72) Erfinder: Scherkenbeck, Jürgen, Dr., D-51381 Leverkusen (DE); Jeschke, Peter, Dr., D-51373 Leverkusen (DE); Lerchen, Hans-Georg, Dr., D-51061 Köln (DE); Hagemann, Hermann, Dr., D-51375 Leverkusen (DE); Harder, Achim, Dr. Dr., D-51109 Köln (DE); Mencke, Norbert, Dr., D-51381 Leverkusen (DE); Plant, Andrew, Dr., D-51519 Odenthal (DE)

(56) Entgegenhaltungen:
- EP-A- 0 382 173
- CHEMICAL ABSTRACTS, vol. 79, no. 3, 23. Juli 1973, Columbus, Ohio, US; abstract no. 19074, V. Z. PLETNEV ET AL 'Theoretical conformational analysisof cyclic octadepsipeptides' Seite 486 ; & KHIM. PRIR. SOEDIN. Nr. 2 , 1973 Seiten 220 - 224
- CHEMICAL ABSTRACTS, vol. 66, no. 7, 13. Februar 1967, Columbus, Ohio, US; abstract no. 29078, V. T. IVANOV ET AL 'Synthetic and natural cyclodepsipeptides' Seite 2795 ; & PEPTIDES, PROC. EUROPEAN SYMP., 6TH, ATHENS 1963 Seiten 337 - 350
- CHEMISCHE BERICHTE Bd. 101, Nr. 5 , 1968 , WEINHEIM DE Seiten 1532 - 1539 G. LOSSE ET AL 'Synthese von Stereoisomeren des Enniatins B'
- TETRAHEDRON LETTERS. Nr. 46 , 1977 , OXFORD GB Seiten 4049 - 4050 M. KANAOKA ET AL 'Synthesis of bassianolide'
- CHEMICAL ABSTRACTS, vol. 119, no. 21, 22. November 1993, Columbus, Ohio, US; abstract no. 224429, TOORU SASAKI ET AL 'Isolation and anthelmintic cyclic depsipeptides from nonspore-forming microorganism' Seite 871 ; & JP-A-05 170 749 (MEIJI SEIKA CO.)

## Beschreibung

Die vorliegende Erfindung betrifft neue Octacyclodepsipeptide sowie mehrere Verfahren zu deren Herstellung und ihre Verwendung als Endoparasiticide,

Aus der EP-OS 0 382 173 ist ein cyclisches Depsipeptid mit der Bezeichnung PF 1022 bekannt. Die Verbindung besitzt anchelminthische Wirkung. Bei niedrigen Aufwandmengen läßt die Wirksamkeit allerdings in manchen Fällen zu wünschen übrig.

Pletnev et. al. (Khim. Prir. Soedin: 1973 (2), 220-224; entsprechend Chemical Abstracts 79 (1973) 19074e) beschreiben die Konformationsanalyse eines Cyclooctadepsipeptides.

Ivanov et. al. (Peptides, Proc. European Symp., 6th, Athens 1963, 337-350, entsprechend Chemical Abstracts 66 (1967) 29078e) untersuchten die Synthese und Strukturaufklärung der natürlichen Cyclodepsipeptide Enniatin A und B, Sporodesmolid I und II und Valinomycin.

Losse et. al. (Chem. Ber. 101 (1968) 1532-1539) beschreiben die Synthese von Stereoisomeren des Enniatins B.

Kanaoka et. al. (Tetrahedron Letters 46 (1977) 4049-4050 beschreiben die Synthese von Bassianolid, einen insektiziden Cyclodepsipeptid.

JP (Kokai) 05 170 749-(entsprechend Chemical Abstracts 119 (1993) 224429k offenbart die anthelmintischen Cyclooctadepsipeptide PF1022B, PF1022C, PF1022D und ihre Gewinnung aus Kulturen eines Mikroorganismus.

Gegenstand der vorliegenden Erfindung sind nun
1. Verbindungen der allgemeinen Formel (I) 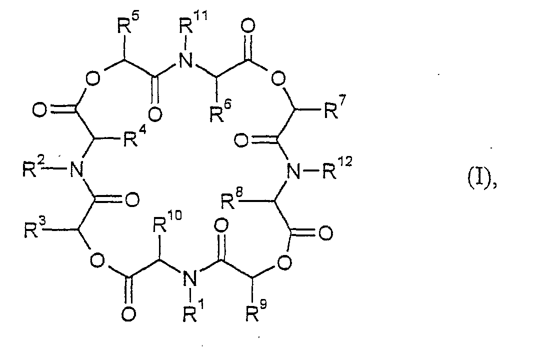 in welcher die Reste R¹ bis R¹² die in folgender Tabelle angegebene Bedeutung haben: 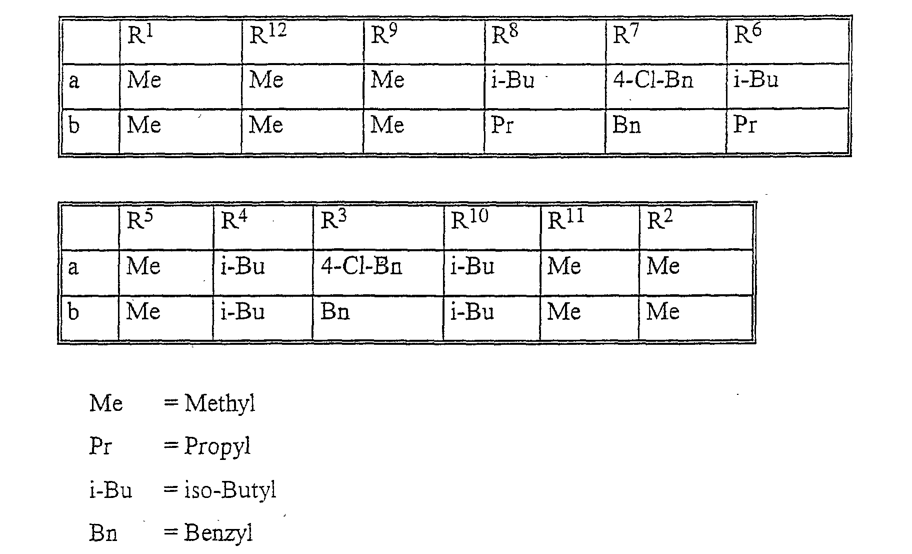 sowie deren Stereoisomere
2. Verfahren zur Herstellung der Verbindungen der Formel (I) 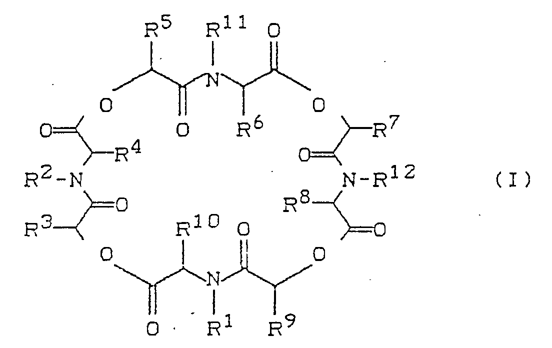 in welcher
   R¹ bis R¹² die oben angegebene Bedeutung haben,
   dadurch gekennzeichnet, daß man
   offenkettige Octadepsipeptide der Formel (II) 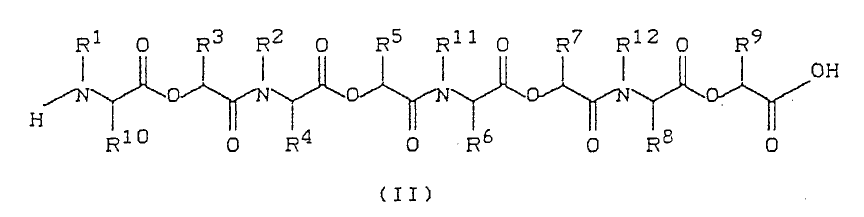 in welcher
   R¹ bis R¹² die oben angegebene Bedeutung haben,
   in Gegenwart eines Verdünnungsmittels und in Gegenwart eines Kopplungsreagenzes cyclisiert;
   In den folgenden Punkten 3. bis 13. werden Verfahren und zwischen produkte zur Herstellung den Verbindungen der Formel (I) beschreiben.
3. Offenkettige Octadepsipeptide der Formel (II) 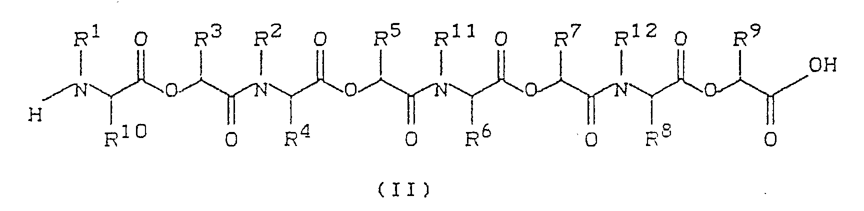 in welcher
   R¹ bis R¹² die oben angegebene Bedeutung haben
   sowie deren Stereoisomere.
4. Verfahren zur Herstellung der offenkettigen Octadepsipeptide der Formel (II) 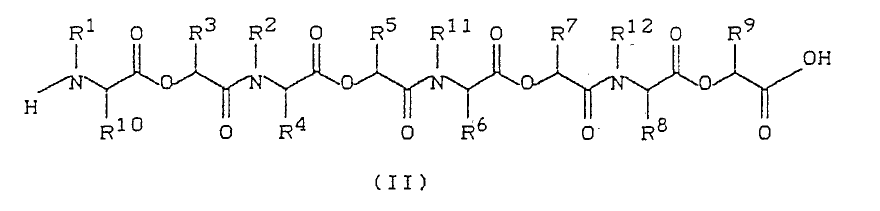 in welcher
   R¹ bis R¹² die oben angegebene Bedeutung haben
   dadurch gekennzeichnet, daß man Verbindungen der Formel (III) 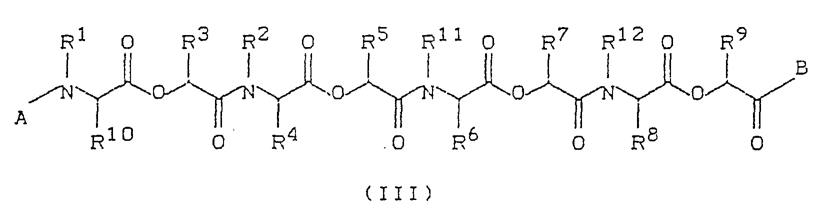 in welcher
   - A: für Benzyl und
   - B: für OH steht und
   - R¹ bis R¹²: die oben angegebene Bedeutung besitzen,
   in Gegenwart eines Verdünnungsmittels und eines Katalysators hydrogenolysiert.
5. Verbindungen der Formel (III) 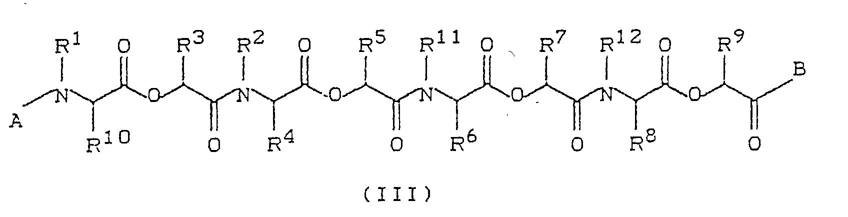 in welcher
   - A: für Benzyl und
   - B: für OH steht und
   - R¹ bis R¹²: die oben angegebene Bedeutung haben
6. Verfahren zur Herstellung der Verbindungen der Formel (III) 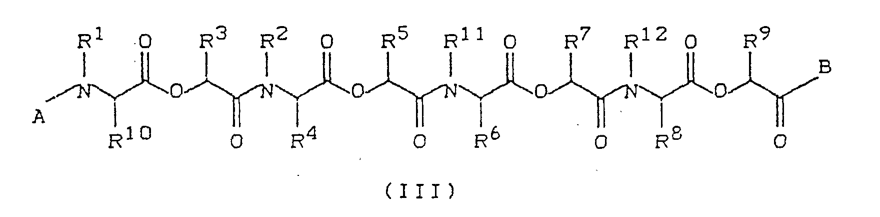 in welcher
   - A: für Benzyl und
   - B: für OH steht und
   - R¹ bis R¹²: die oben angegebene Bedeutung haben,
   dadurch gekennzeichnet, daß man Verbindungen der Formel (IV) 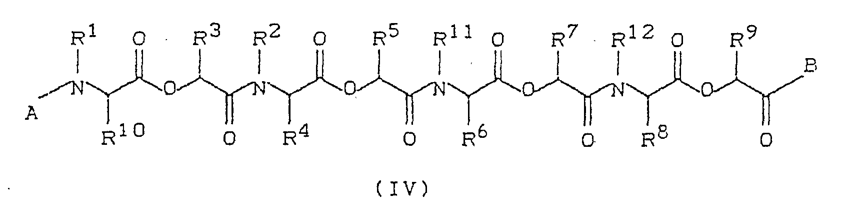 in welcher
   - A: für Benzyl und
   - B: für tert.-Butoxy steht sowie
   - R¹ bis R¹²: die oben angegebene Bedeutung haben,
7. Verfahren zur Herstellung der Verbindungen der Formel (IV) 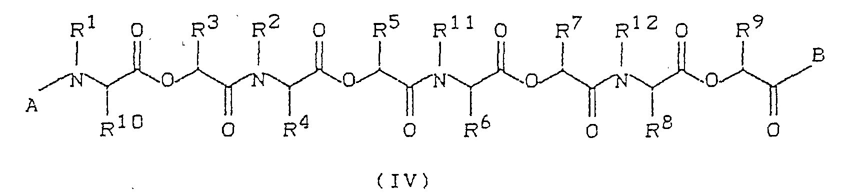 in welcher
   - A: für Benzyl und
   - B: für tert.-Butoxy steht und
   - R¹ bis R¹²: die oben angegebene Bedeutung haben
   sowie deren Stereoisomere, dadurch gekennzeichnet, daß man Tetradepsipeptide der Formel (V) 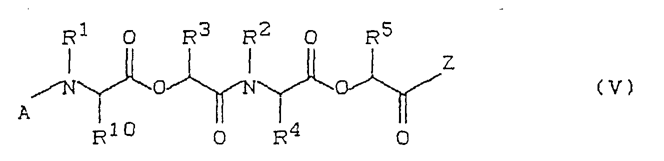 in welcher
   - A: für Benzyl und
   - Z: für OH steht sowie
   - R¹, R², R³, R⁴, R⁵ und R¹⁰: die oben angegebene Bedeutung haben
   und Tetradepsipeptide der Formel (VI) 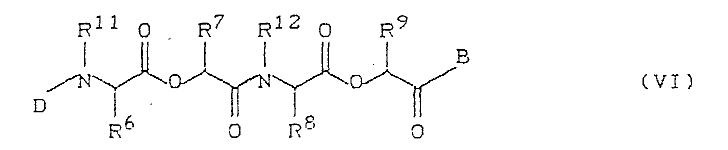 in welcher
   - D: für Wasserstoff und
   - B: für tert.-Butoxy steht sowie
   - R⁶, R⁷, R⁸, R⁹, R¹¹ und R¹²: die oben angegebene Bedeutung haben,
   in Gegenwart eines Verdünnungsmittels und eines Kopplungsreagenzen kondensiert.
8. Tetradepsipeptide der Formel (V) 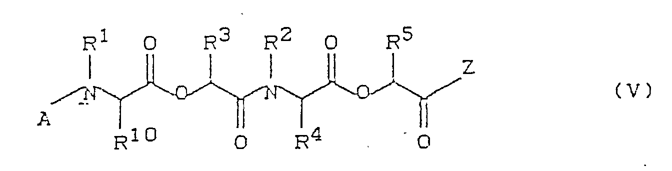 in welcher
   - A: für Benzyl und
   - Z: für OH steht sowie
   - R¹, R², R³, R⁴, R⁵, R¹⁰: die oben angegebene Bedeutung haben.
9. Tetradepsipeptide der Formel (VI) 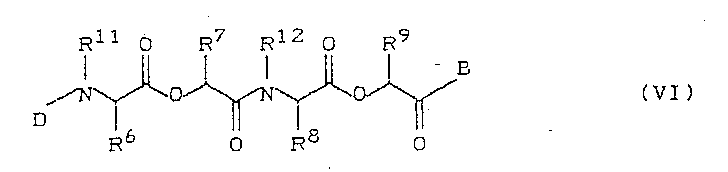 in welcher
   - D: für Wasserstoff und
   - B: für tert.-Butoxy steht sowie
   - R⁶, R⁷, R⁸, R⁹, R¹¹, R¹²: die oben angegebene Bedeutung haben.
10. Verfahren zur Herstellung der Tetradepsipeptide der Formel (V) 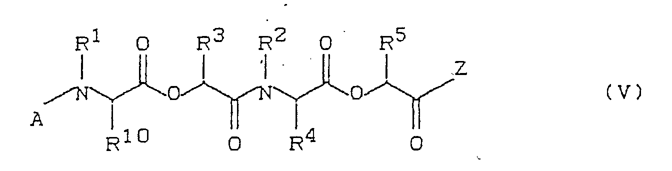 in welcher
   - A: für Benzyl und
   - Z: für OH steht sowie
   - R¹, R², R³, R⁴, R⁵, R¹⁰: die oben angegebene Bedeutung haben,
   dadurch gekennzeichnet, daß man Tetradepsipeptide der Formel (VII) 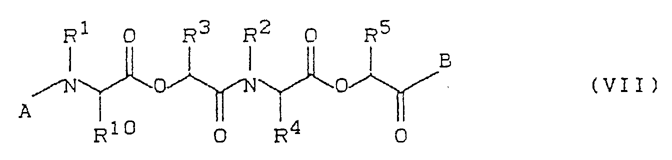 in welcher
   - A: für Benzyl und
   - B: für tert.-Butoxy steht sowie
   - R¹, R², R³, R⁴, R⁵ und R¹⁰: die oben angegebene Bedeutung besitzen,
   in Gegenwart eines Verdünnungsmittels und einer Protonensäure verseift.
11. Verfahren zur Herstellung der Tetradepsipeptide der Formel (VI) 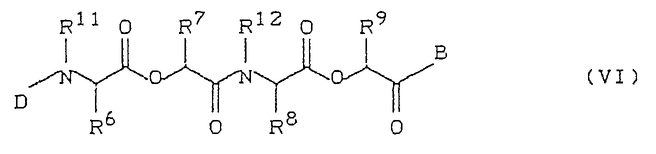 in welcher
   - D: für Wasserstoff und
   - B: für tert.-Butoxy steht sowie
   - R⁶, R⁷, R⁸, R⁹, R¹¹, R¹²: die oben angegebene Bedeutung haben,
   dadurch gekennzeichnet, daß man Tetradepsipeptide der Formel (VII) 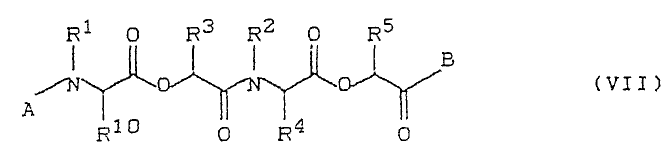 in welcher
   - A: für Benzyl und
   - B: für tert.-Butoxy steht sowie
   - R¹, R², R³, R⁴, R⁵ und R¹⁰: die oben angegebene Bedeutung besitzen,
   in Gegenwart eines Verdünnungsmittels und eines Katalysators hydrogenolysiert.
12. Tetradepsipeptide der Formel (VII) 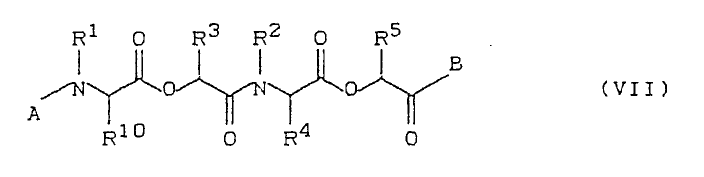 in welcher
   - A: für Benzyl und
   - B: für tert.-Butoxy steht sowie
   - R¹, R², R³, R⁴, R⁵, R¹⁰: die oben angegebene Bedeutung haben.
13. Verfahren zur Herstellung der Tetradepsipeptide der Formel (VII) 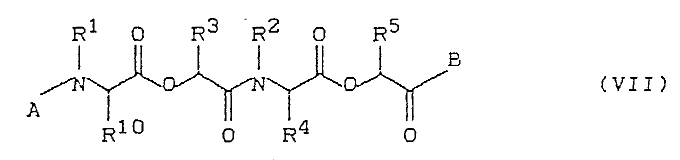 in welcher
   - A: für Benzyl und
   - B: für tert.-Butoxy steht sowie
   - R¹, R², R³, R⁴, R⁵, R¹⁰: die oben angegebene Bedeutung haben,
   dadurch gekennzeichnet, daß man Didepsipeptide der Formel (VIII) 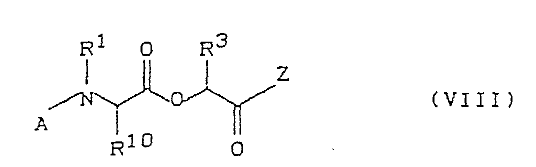 in welcher
   - A: für Benzyl und
   - Z: für OH steht sowie
   - R¹, R³ und R¹⁰: die oben angegebene Bedeutung besitzen und
   Didepsipeptide der Formel (IX) 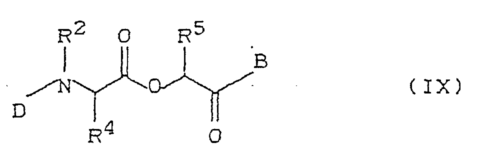 in welcher
   - D: für Wasserstoff und
   - B: für tert.-Butoxy steht sowie
   - R², R⁴ und R⁵: die oben angegebene Bedeutung besitzen,
   in einem Verdünnungsmittel in Gegenwart eines Kopplungsreagenzes kondensiert.

Schließlich wurde gefunden, daß die neuen Octacyclodepsipeptide der Formel (I) sowie deren SäureadditionsSalze und Metallsalz-Komplexe sehr gute anthelminthische Eigenschaften besitzen und bevorzugt im Bereich der Veterinärmedizin eingesetzt werden können. Überraschenderweise zeigen die erfindungsgemäßen Stoffe bei der Bekämpfung von Wurmerkrankungen eine bessere Wirksamkeit konstitutionell ähnliche, vorbekannte Verbindungen gleicher Wirkungsrichtung,

Gegebenenfalls substituiertes Alkyl allein oder als Bestandteil eines Restes in den allgemeinen Formeln bedeutet geradkettigesoderverzweigtesAlkylmitvorzugsweise 1 bis 9, insbesondere 1 bis 5 Kohlenstoffatomen. Beispielhaft und vorzugsweise seien gegebenenfalls substituiertes Methyl, Ethyl, n- und i-Propyl, n-, i- und t-Butyl, genannt.

Gegebenenfalls substituiertes Alkenyl allein oder als Bestandteil eines Restes in den allgemeinen Formeln bedeutet geradkettiges oder verzweigtes Alkenyl mit vorzugsweise 2 bis 20, insbesondere 2 bis 8 Kohlenstoffatomen, Beispielhaft und vorzugsweise seien gegebenenfalls substituiertes Ethenyl, Propenyl-(1), Propenyl-(2) und Butenyl-(3) genannt.

Gegebenenfalls substituiertes Cycloalkyl in den allgemeinen Formeln bedeutet mono-, bi- und tricyclisches Cycloalkyl mit vorzugsweise 3 bis 10, insbesondere 3, 5 oder 6 Kohlenstoffatomen. Beispielsweise und vorzugsweise seien gegebenenfalls substituiertes Cyclopropyl, Cyclobutyl, Cyclopentyl, Cyclohexyl und Cycloheptyl genannt.

Gegebenenfalls substituiertes Alkoxy in den allgemeinen Formeln bedeutet geradkettiges oder verzweigtes Alkoxy mit vorzugsweise 1 bis 6, insbesondere 1 bis 4 Kohlenstoffatomen. Beispielhaft und vorzugsweise seien gegebenenfalls substituiertes Methoxy, Ethoxy, n- und i-Propoxy und n-, o- und t-Butoxy genannt.

Gegebenenfalls substituiertes Alkylthio in den allgemeinen Formeln bedeutet geradkettiges oder verzweigtes Alkylthio mit vorzugsweise 1 bis 6, insbesondere 1 bis 4 Kohlenstoffatomen. Beispielhaft und vorzugsweise seien gegebenenfalls substituiertes Methylthio, Ethylthio, n-und i-Propylthio, n-, o- und t-Butylthio genannt.

Halogenalkyl in den allgemeinen Formeln enthält 1 bis 4, insbesondere 1 oder 2 Kohlenstoffatome und vorzugsweise 1 bis 9, insbesondere 1 bis 5 gleiche gleiche oder verschiedene Halogenatome, wobei als Halogenatome vorzugsweise Fluor, Chlor und Brom, insbesondere Fluor und Chlor stehen. Beispielhaft seien Trifluormethyl, Chlordi-fluormethyl, 2,2,2-Trifluorethyl und Pentafluormethyl, Perfluor-t-butyl genannt.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet gegebenenfalls im Arylteil und/oder Alkylteil substituiertes Aralkyl mit vorzugsweise 6 oder 10, insbesondere 6 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls Heteroaryl allein oder als Bestandteil eines Restes bedeutet in den allgemeinen Formeln 5- bis 7-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien gegebenenfalls substituiertes Furyl, Thiophenyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,2,3-, 1,3,6- und 1,2,6-Oxazinyl, Oxepinyl, Thiepinyl und 1,2,4-Diazepinyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Difluormethyl, Trifluormethyl; Hydorxy; Halogen, vorzugsweise Fluor, Chlor, Brom, insbesondere Fluor, Chlor, Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, n-und i-Propylamino und Methyl-n-Butylamino; Acylreste wie Carboxyl; Carboxy mit vorzugsweise 2 bis 4, insbesondere 2 oder 3 Kohlenstoffatomen, wie Carbomethoxy und Carboethoxy; Alkylsulfinyl mit 1 bis 4, insbesondere 1 bis 2 Kohlenstoffatomen, Halogenalkylsulfinyl mit 1 bis 4, insbesondere 1 bis 2 Kohlenstoffatomen und 1 bis 5 Halogenatomen wie Trifluormethylsulfinyl; Sulfonyl (-SO₃H); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit 1 bis 4, insbesondere 1 bis 2 Kohlenstoffatomen und 1 bis 5 Halogenatomen wie Trifluormethylsulfonyl, Perfluor-t,n,s-butylsulfonyl; Arylsulfonyl mit vorzugsweise 6 oder 10 Arylkohlenstoffatomen, wie Phenylsulfonyl; Acyl, Aryl, Aryloxy, Heteroaryl, Heteroaryloxy, die ihrerseits einen der oben genannten Substituenten tragen können sowie der Formiminorest

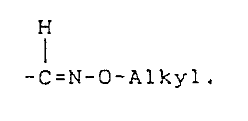

Bei Verfahren 2 werden Octadepsipeptide in Gegenwart von Verdünnungsmitteln und geeigneten Kopplungsreagenzien cyclisiert.

Als Kopplungsreagenzien eignen sich alle Verbindungen, die zur Knüpfung einer Amidbindung geeignet sind (vgl. z.B.: Houben-Weyl, Methoden der organischen Chemie, Band 15/2; Bodanszky et al., Peptide Synthesis 2nd ed. (Wiley & Sons, New York 1976).

Vorzugsweise kommen folgende Reagenzien und Methoden in Frage, Aktivestermethode mit Pentafluorphenol (Pfp), N-Hydroxysuccinimid, 1-Hydroxybenzotriazol, Kupplung mit Carbodiimiden, wie Dicyclohexylcarbodiimid oder N'-(3-Dimethylaminopropyl)-N-ethyl-carbodiimid (Ebc) sowie die gemischte Anhydrid-Methode oder die Kopplung mit Phosphoniumreagenzien, wie Benzotriazol-1-yl-oxy-tris(dimethylaminophosphonium)-hexafluorophosphat (BOP), Bis(2-oxo-3-oxazolidinyl)-phosphoniumsäurechlorid (BOP-Cl), oder mit Phosphonsäureesterreagentien, wie Cyanphosphonsäurediethylester (DEPc) und Diphenylphospharylazid (DPPA).

Besonders bevorzugt ist die Kupplung mit Bis(2-oxo-3-oxazolidinyl)-phosphoniumsäurechlorid (BOP-Cl) und N'-(3-Dimethylaminopropyl)-N-ethylcarbodiimid (EDC) in Gegenwart von 1-Hydroxybenzotriazol (HOBt).

Die Umsetzung erfolgt bei Temperaturen von 0 - 150°C, bevorzugt bei 20 - 100°C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid,

Die Verbindungen der Formeln (II) und die Kopplungsreagenzien werden im Verhältnis 1:1 bis 1:1,5 zueinander eingesetzt. Bevorzugt ist ein etwa äquimolares Verhältnis.

Nach erfolgter Umsetzung wird das Verdünnungsmittel abdestilliert und die Verbindungen der Formel (I) in üblicher Weise, z.B. chromatographisch, gereinigt.

Die Reaktion gemäß Verfahren 4 wird unter Verwendung von Hydrierungsmitteln durchgeführt.

Als bevorzugtes Hydrierungsmittel sei Wasserstoff in Gegenwart der üblichen Hydrierungskatalysatoren, wie z.B. Raney-Nickel, Palladium und Platin genannt.

Das Verfahren wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Methyl-tert.-butylether, Glykoldimethylether und Diglykoldimethylether, Tetrahydrofuran undDioxan, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid; ferner auch Alkohole wie Methanol, Ethanol, Propanol, Isopropanol, Butanol, Isobutanol, sec.-Butanol, tert.-Butanol, Pentanol, Isopentanol, sec.-Pentanol und tert.-Pentanol sowie auch Wasser.

Die Reaktionstemperaturen können bei dem erfindungsgemäßen Verfahren in einem größeren Bereich variiert werden. Im allgemeinen arbeitet man bei Temperaturen zwischen -20°C und +200°C, vorzugsweise bei Temperaturen zwischen 0°C und 120°C.

Das erfindungsgemäße Verfahren wird im allgemeinen unter Normaldruck durchgeführt. Es ist jedoch auch möglich, unter erhöhtem, im allgemeinen zwischen 10 und 100 bar, zu arbeiten.

Die Reaktion gemäß Verfahren 6 wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Mehylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid,

Die Reaktion wird in Gegenwart von anorganischen oder organischen Protonensäuren durchgeführt.

Als solche seien z.B. genannt: Salzsäure, Schwefelsäure, Trifluoressigsäure, Essigsäure, Ameisensäure.

Die Umsetzung erfolgt bei Temperaturen zwischen -20 und +50°C, vorzugsweise zwischen -10 und +20°C bei Normaldruck oder erhöhtem Druck. Es wird bevorzugt bei Normaldruck gearbeitet.

Die Reaktion gemäß Verfahren 8 wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid,

Die Reaktion wird in Gegenwart von anorganischen oder organischen Säureakzeptoren durchgeführt.

Als solche seien z.B. genannt:

Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw, -ethylat, ferner aliphatische, aromatische oder heterocyclische Amine, beispielsweise Triethylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO), Ethyl-diisopropylamin.

Die Umsetzung erfolgt bei Temperaturen zwischen 10 und 150°C, vorzugsweise zwischen 20 bis 100°C bei Normaldruck oder erhöhtem Druck. Es wird bevorzugt bei Normaldruck gearbeitet.

Verfahren 11 wird durchgeführt, wie weiter oben bei der Durchführung von Verfahren 6 angegeben.

Das unter 12 beschriebene erfindungsgemäße Verfahren wird wie bei Verfahren 4 angegeben durchgeführt.

Verfahren 14 wird durchgeführt wie weiter oben bei der Durchführung von Verfahren 8 angegeben.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobethrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt; Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefugt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methyl-pyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol,

Fettsäuren wie z.B. Ölsäure und ihre Gemische.

Als hydrophile Phase seien genannt: Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden, Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungsund Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder linerares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffe, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1-10 Gewichtsprozent.

Zubereitungen die vor Anwendung verdünnt werden, enthalten den Wirkstoff in Konzentrationen von 0,5-90 Gew.-%, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Haemonchus contortus / Schaf

Experimentell mit Heamonchus contortus infizierte Schafe wurden nach Ablauf der Präpatenzzeit des Parasiten behandelt. Die Wirkstoffe wurden als reiner Wirkstoff in Gelatinekapseln oral appliziert (p.o) oder als Lösung intravenös injiziert (i.v.).

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Die Herstellung der erfindungsgemäßen Wirkstoffe geht aus der nachfolgenden Beispielen hervor.

### Herstellungsbeispiele

1. Herstellung der Verbindungen der Formel (I) gemäß Verfahren 2.
Zu einer Lösung der Verbindung der Formel II (0,104 mmol) und Hünig-Base (0,258 mmol) in Dichlormethan (100 ml) wurde bei 0°C BOP-C1 (0,124 mmol) zugegeben und 24 h bei Raumtemperatur nachgerührt. Nach dieser Zeit wurden dieselben Mengen BOP-Cl und Base zugesetzt und weitere 24 h gerührt. Die Lösung wurde zweimal mit ges. Natriumhydrogencarbonatlösung gewaschen, über Natriumsulfat getrocknet und eingeengt. Der Rückstand wurde säulenchromatografisch mit dem Laufmittel Cyclohexan-Ethylacetat 2:1 gereinigt.
Es wurden Verbindungen der Formel (I) erhalten, in welcher die Substituenten die folgende Bedeutung haben: 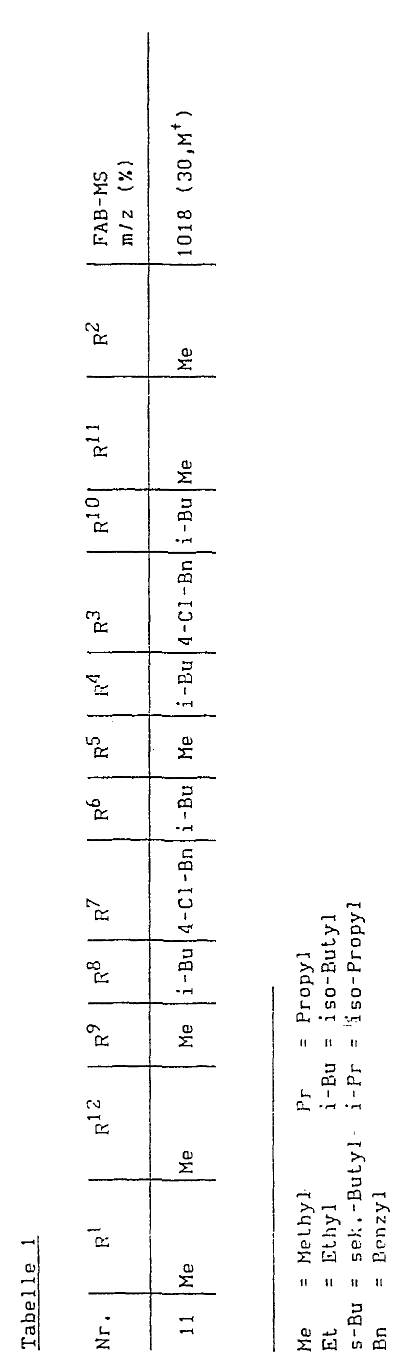 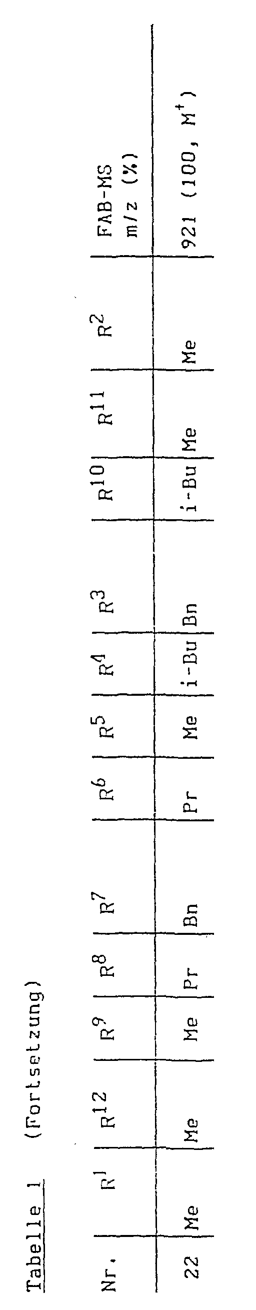
2. Herstellung der Verbindungen der Formel (II) gemäß Verfahren 4.
Eine Lösung der Verbindung der Formel III (1.222 mmol) in Ethanol (50 ml) wurde in Gegenwart von Pd(OH)₂/C (20 %; 200 mg) bis zur Beendigung der Wasserstoffaufnahme hydriert (ca. 2h). Nach Abfiltrieren des Katalysators fiel reine Verbindung der Formel II an, die ohne zusätzliche Reinigung weiter umgesetzt wurde.
Gemäß dieser Vorschrift wurden Verbindungen der Formel (II) erhalten, in welcher die Substituenten die Bedeutung gemäß Tabelle 1 haben. 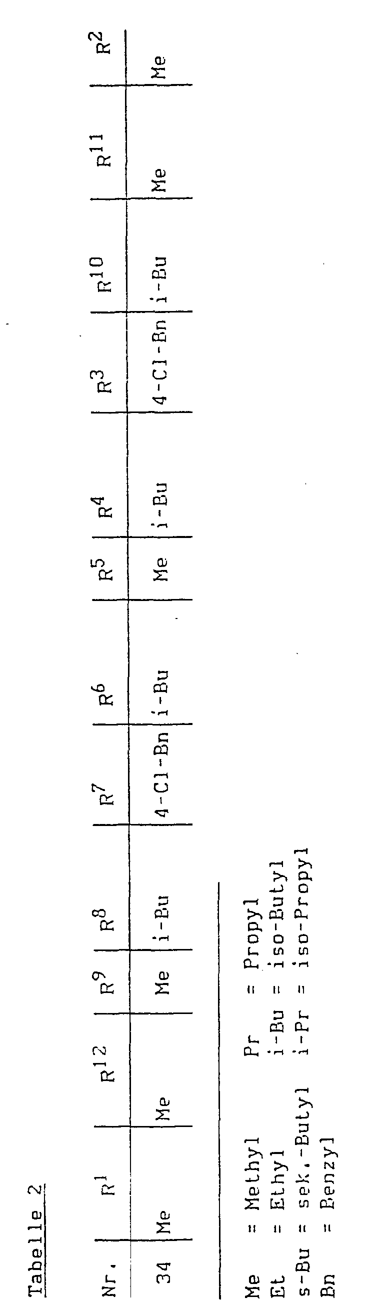 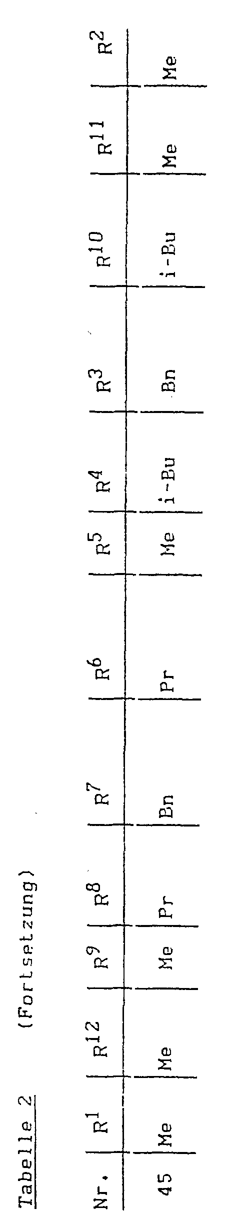
4. Herstellung der Verbindungen der Formel (III) gemäß Verfahren 6
In eine Lösung des tert.-Butylesters der Formel (IV) (1.609 mmol) in Dichlormethan (40 ml) wurde bei 0°C 1,5 h HCl-Gas eingeleitet. Anschließend wurde auf Raumtemperatur aufgewärmt und 12 h nachgerührt. Die Lösung wurde einrotiert und im Hochvakuum getrocknet. Der Rückstand wurde ohne weitere Reinigung umgesetzt.
Analog wurden Verbindungen der Formel (III) erhalten, in welcher die Substituenten die folgende Bedeutung haben: 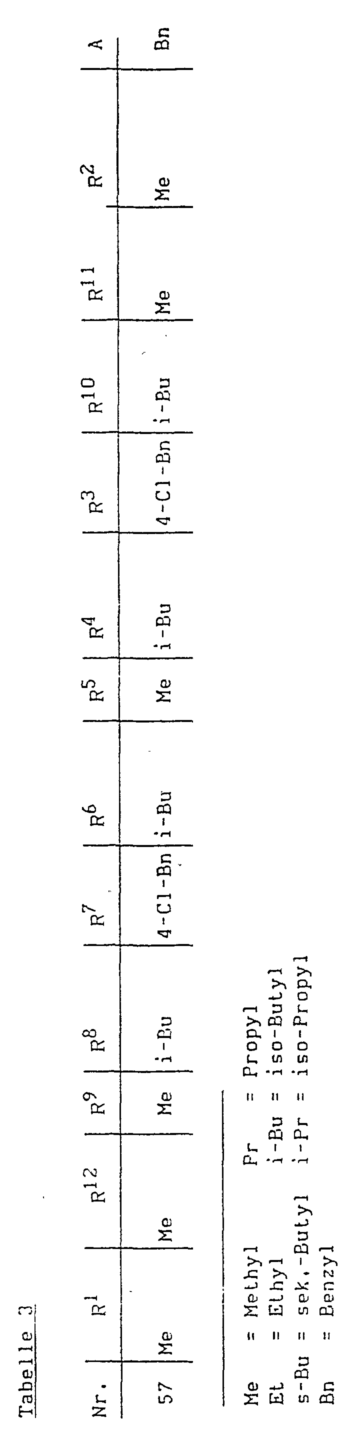 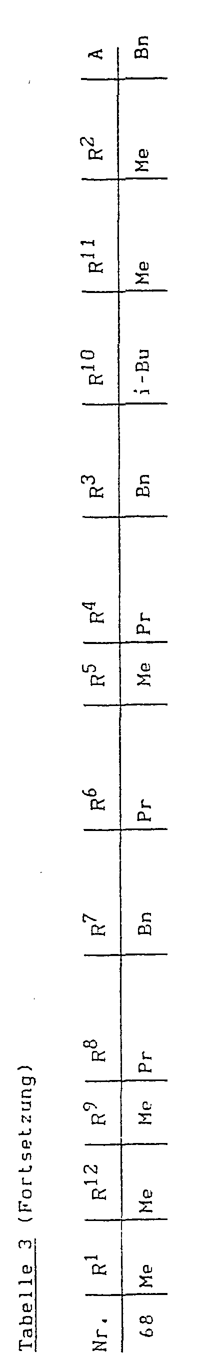
5. Herstellung der Verbindungen der Formel (IV) gemäß Verfahren 8
Zu einer Lösung der Tetradepsipeptide der Formel (VI) und (V) je (2.52 mmol), in Dichlormethan (15 ml) wurde bei 0°C eine Lösung von Ethyldiisopropylamin (0,912 mmol) und BOP-Cl (0,438 mmol) zugegeben. Es wurde 1 h bei 0°C und 1,5 h bei Raumtemperatur nachgerührt, mit 20 ml Dichlormethan verdünnt, zweimal mit wenig Wasser gewaschen, über Na₂SO₄ getrocknet und eingeengt. Der Rückstand wurde an Kieselgel mit dem Laufmittel Cyclohexan-t-BuOMe = 2 : 1 gereinigt.
Gemäß dieser Vorschrift wurden Verbindungen der Formel (IV) erhalten, in welcher die Substituenten die-folgende Bedeutung haben: 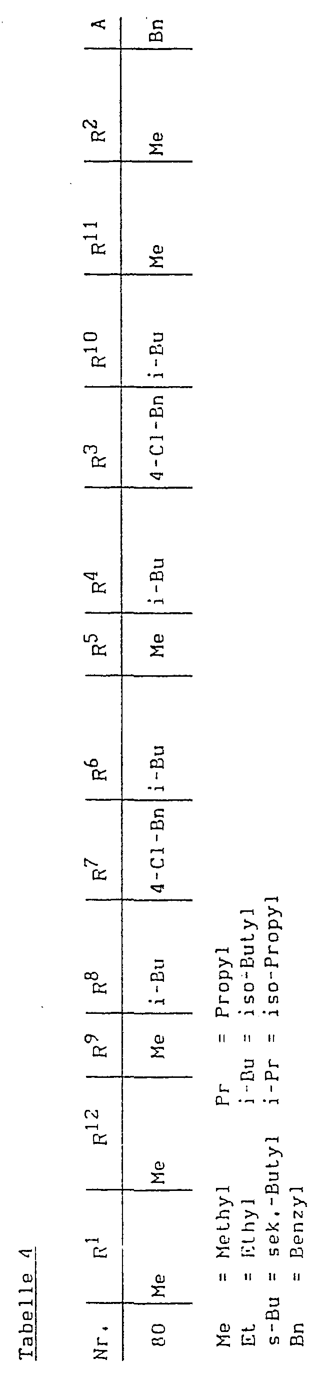 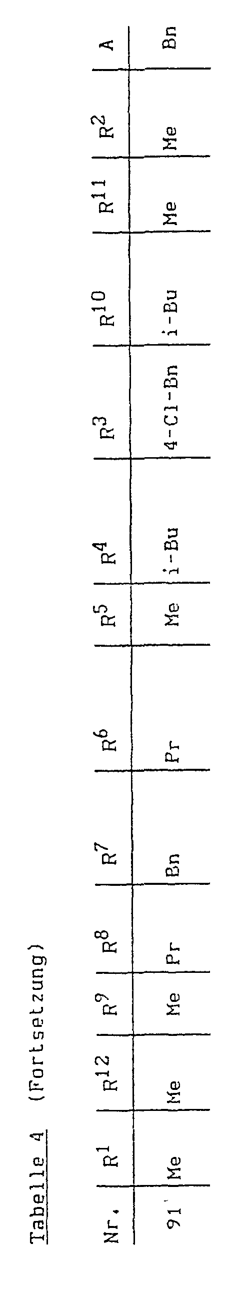
6. Herstellung der Verbindungen der Formel (V) gemäß Verfahren 11
In eine Lösung des Tetradepsipeptids mit der Formel (VII) (2.848 mmol) in Dichlormethan (50 ml) wurde bei 0°C HCl-Gas 2 h eingeleitet.
Anschließend wurde 8 h bei Raumtemperatur nachgerührt, eingeengt und im Hochvakuum getrocknet. Der Rückstand wurde ohne weitere Reinigung eingesetzt.
Gemäß dieser Vorschrift wurden die folgenden Verbindungen der Formel (V) erhalten, in welcher die Substituenten die folgende Bedeutung haben:

**Tabelle 5**

| Nr. | R¹ | R¹² | R⁹ | R⁸ | R⁷ | R¹⁰ | A | Z |
|---|---|---|---|---|---|---|---|---|
| 100 | Me | Me | Me | i-Bu | 4-Cl-Bn | i-Bu | Bn | OH |
| 105 | Me | Me | Me | Pr | Bn | Pr | Bn | OH |
| Me = Methyl Pr = Propyl | | | | | | | | |
| Et = Ethyl i-Bu = iso-Butyl | | | | | | | | |
| s-Bu = sek.-Butyl i-Pr = iso-Propyl | | | | | | | | |
| Bn = Benzyl | | | | | | | | |

7. Herstellung der Verbindungen der Formel (VI) gemäß Verfahren 12
Eine Lösung des Tetradepsipeptids mit der Formel (VII) (9.53 mmol) -in Ethanol (37 ml) wurde mit Pd(OH)₂/C (20 %) (0,6 g) versetzt und ca. 3 h bei Raumtemperatur und Normaldruck hydriert. Das Reaktionsgemisch wurde filtriert, eingeengt und der Rückstand an Kieselgel mit dem Laufmittel t-BuOMe-Cyclohexan-Ethanol = 1:1:0,5 getrennt.
Gemäß diesem Verfahren wurden die folgenden Verbindungen der Formel (VI) erhalten, in welcher die Substituenten die folgende Bedeutung haben:

**Tabelle 6**

| Nr. | R¹ | R¹² | R⁹ | R⁸ | R⁷ | R¹⁰ | D | B |
|---|---|---|---|---|---|---|---|---|
| 114 | Me | Me | Me | i-Bu | 4-Cl-Bn | i-Bu | H | t-Bu-O- |
| 119 | Me | Me | Me | Pr | Bn | Pr | H | t-Bu-O- |
| Me = Methyl Pr = Propyl Et = Ethyl iPr = iso-Propyl Bu = Butyl sBu = sek.-Butyl Bn = Benzyl iBu = iso-Butyl | | | | | | | | |

8. Herstellung der Verbindungen der Formel (VII) gemäß Verfahren 14
Eine auf 0°C gekühlte Lösung des Didepsipeptids IX (22,9 mmol) und des Didepsipeptids VIII in Dichlormethan (80 ml) wurde mit Diisopropylethylamin (57,3 mmol) und BOP-Cl (29,8 mmol) versetzt, 1 h bei 0°C und 1 h bei Raumtemperatur gerührt. Nach Abfiltrieren des Niederschlages wurde die Lösung mit Dichlormethan verdünnt, dreimal mit wenig Wasser gewaschen, über Natriumsulfat getrocknet und eingeengt. Der Rückstand wurde an Kieselgel mit dem Laufmittel Cyclohexan-Ethylacetat = 15:1 getrennt.
Gemäß dieser Vorschrift wurden die folgenden Verbindungen der Formel VII erhalten, in welcher die Substituenten die folgende Bedeutung haben.

**Tabelle 7**

| Nr. | R¹ | R¹² | R⁹ | R⁸ | R⁷ | R¹⁰ | A | C |
|---|---|---|---|---|---|---|---|---|
| 128 | Me | Me | Me | i-Bu | 4-Cl-Bn | i-Bu | H | t-BuO |
| 133 | Me | Me | Me | Pr | Bn | Pr | H | t-BuO |

9. Herstellung der Verbindungen der Formel (VIII) gemäß Verfahren 17
In eine Lösung des Didepsipeptids der Formel (X) (46,0 mmol) in Dichlormethan (470 ml) wurde bei 0°C über 2 h HCl-Gas eingeleitet. Bei Raumtemperatur wurde 24 h nachgerührt. Der Ansatz wurde eingeengt und der Rückstand im Hochvakuum getrocknet, Der in Wasser gelöste Rückstand wurde in eine Suspension eines basischen Ionenaustauschers (16.7 g) in 50 ml Wasser eingetropft, 3 h gerührt, abfiltriert und eingeengt. Nach Trocknung im Hochvakuum fiel ein amorphes Pulver an, das ohne weitere Reinigung umgesetzt wurde.
Analog wurden die Verbindungen der Formel (VIII) erhalten.
10. Herstellung der Verbindungen der Formel (IX) gemäß Verfahren 18
Eine Lösung des Didepsipeptids (XI) (60 mmol) in Ethanol (163 ml) wurde mit 2,91 g Pd(OH)₂/C (20 %) versetzt und ca, 6 h bei Normaldruck hydriert. Nach Abfiltrieren des Katalysators wurde mit Ethanol nachgewaschen, eingeengt und im Hochvakuum getrocknet. Der Rückstand wurde an Kieselgel mit dem Laufmittel Cyclohexan-Ethylacetat = 3:1 getrennt.
Gemäß dieser Vorschrift wurden die Verbindungen der Formel (IX) erhalten, in welcher die Substituenten die folgende Bedeutung haben:

**Tabelle 9**

| Nr. | R¹² | R⁸ | R⁷ | D | B |
|---|---|---|---|---|---|
| 148 | Me | i-Bu | 4-Cl-Bn | H | t-BuO |
| 154 | Me | Pr | Bn | H | t-BuO |

11. Herstellung der Verbindungen der Formel (X) gemäß Verfahren 21
Das Caesiumsalz der Aminocarbonsäure XII (0,212 mol) wurde in 530 ml Dimethylsulfoxid bei Raumtemperatur vorgelegt und mit der Chlorcarbonsäure XIII (0,212 mol) versetzt. Es wurde 20 h bei Raumtemperatur gerührt, in ges. Kochsalzlösung eingegossen und viermal mit Ethylacetat extrahiert, Die vereinigten organischen Extrakte wurden einmal mit wenig Wasser gewaschen, über Natriumsulfat getrocknet und eingeengt. Der Reinigung des Rückstandes wurde säulenchromatografisch mit dem Laufmitel Cyclohexan-Ethylacetat = 6:1 durchgeführt,
Gemäß dieser Vorschrift wurden die folgenden Verbindungen der Formel (X) erhalten.
12. Herstellung der Verbindungen der Formel (XI) gemäß Verfahren 22
Die Aminosäure der Formel XIV (0,212 mol) wurde in 1000 ml Ethanol und 100 ml Wasser gelöst, mit einer 20 %igen Caesiumcarbonatlösung (200 ml) versetzt und 5 h bei Raumtemperatur gerührt. Anschließend wurde eingeengt, zweimal mit je 250 ml DMF codestilliert und über Nacht bei 80°C im Hochvakuum getrocknet, 0,212 mol dieses Caesiumsalzes wurden in 530 ml Dimethylsulfoxid vorgelegt, bei Raumtemperatur mit 0,212 mol der Chlorcarbonsäure XV versetzt und 20 h bei Raumtemperatur gerührt. Die Lösung wurde in gesättigte Kochsalzlösung eingegossen, viermal mit Ethylacetat extrahiert, über Natriumsulfat getrocknet und eingeengt. Der Rückstand wurde säulenchromatographisch mit dem Laufmittel Cyclohexan-Ethylacetat = 100:1 gereinigt.

## Patentansprüche

1. Verbindungen der allgemeinen Formel (I) 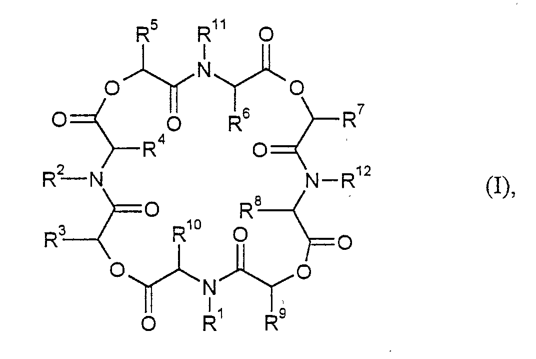 in welcher die Reste R¹ bis R¹² die in folgender Tabelle angegebene Bedeutung haben: 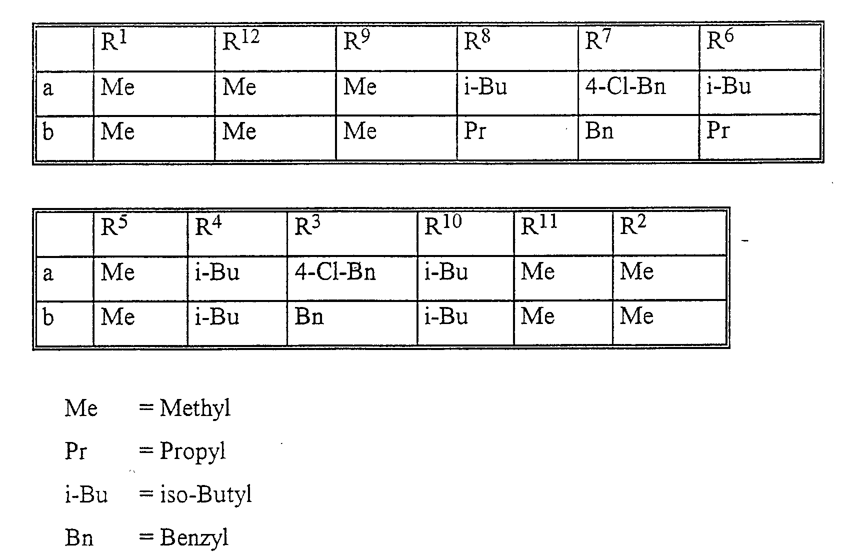 sowie deren Stereoisomere.

2. Verfahren zur Herstellung der Verbindungen der Formel (I) gemäß Anspruch 1 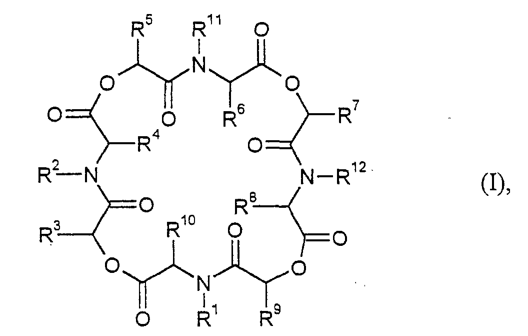 in welcher die Reste R¹ bis R¹² die in Anspruch 1 angegebene Bedeutung haben,
**dadurch gekennzeichnet, daß** man offenkettige Octadepsipeptide der Formel (II) 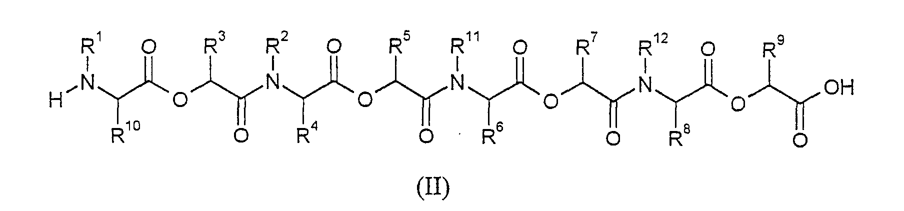 in welcher
R¹ bis R¹² die oben angegebene Bedeutung haben
in Gegenwart eines Verdünnungsmittel und in Gegenwart eines Kupplungsreagenzes cyclisiert.

3. Endoparasitizide Mittel, **gekennzeichnet durch** einen Gehalt an mindestens einer Verbindung der Formel (I) gemäß Anspruch 1.

4. Verwendung von Verbindungen der Formel (I) gemäß Anspruch 1 zur Herstellung von endoparasitiziden Mitteln.

## Claims

1. Compounds of the general formula (I) 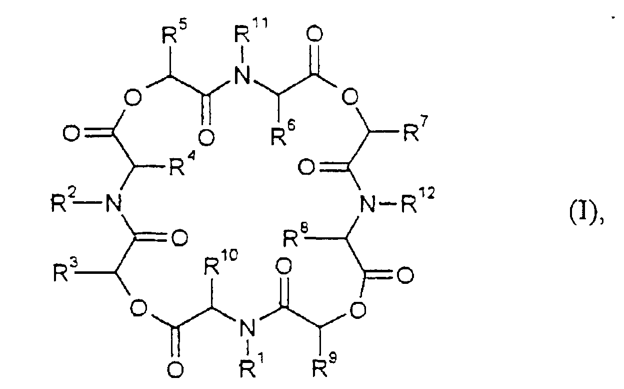 in which the radicals R¹ to R¹² possess the meaning given in the following table: 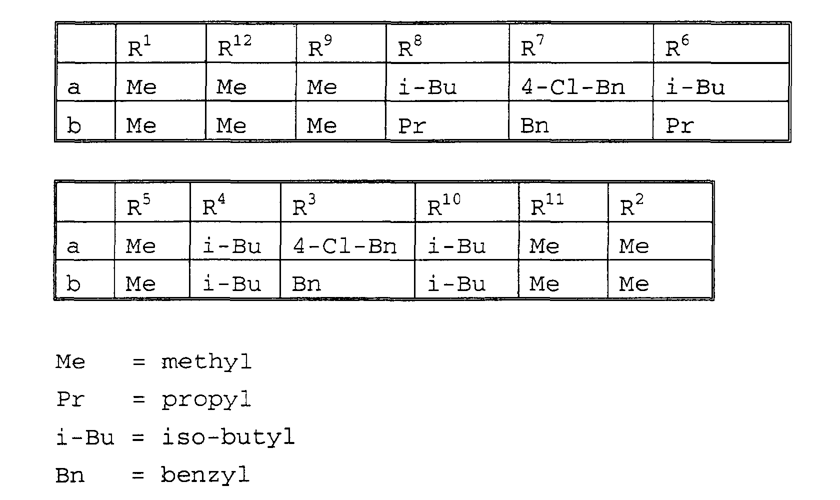 and stereoisomers thereof.

2. Process for the preparation of the compounds of the formula (I) according to Claim 1 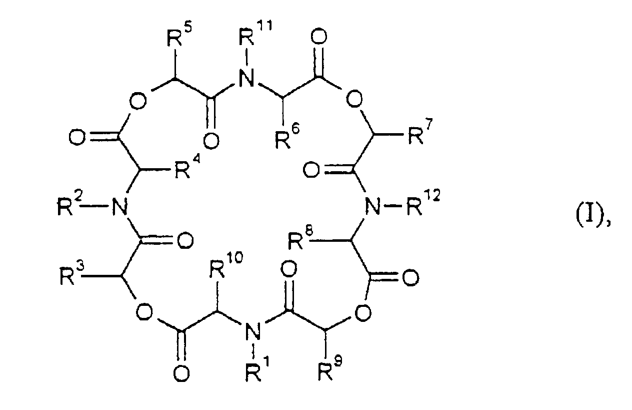 in which the radicals R¹ to R¹² possess the meaning given in Claim 1,
**characterized in that** open-chain octadepsipeptides of the formula (II) 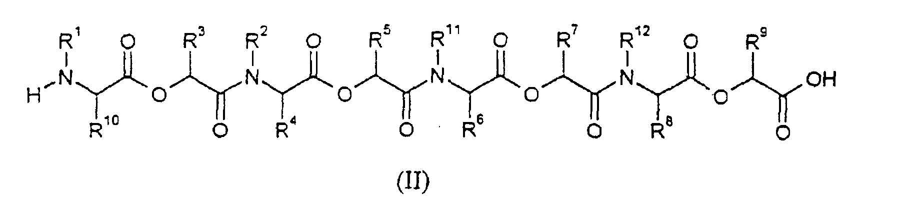 in which
R¹ to R¹² possess the meaning given above
are cyclized in the presence of a diluent and in the presence of a coupling reagent.

3. Endoparasiticidal compositions, **characterized in that** they contain at least one compound of the formula (I) according to Claim 1.

4. Use of compounds of the formula (I) according to Claim 1 for the preparation of endoparasiticidal compositions.

## Revendications

1. Composés de formule générale (I) 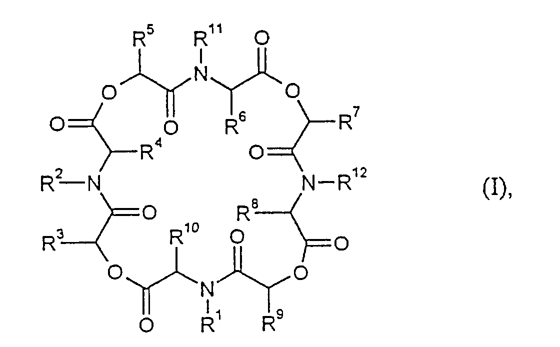 dans laquelle les restes R¹ à R¹² ont la définition indiquée dans le tableau suivant : 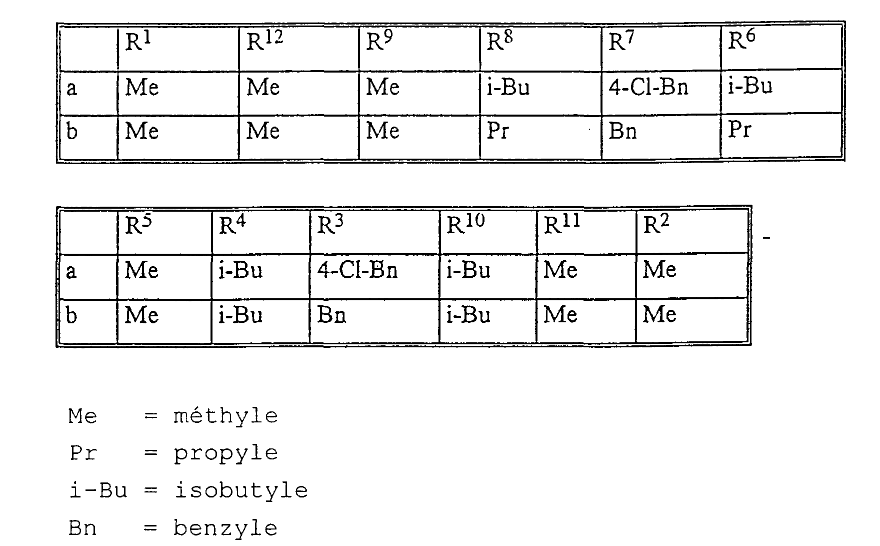 ainsi que leurs stéréo-isomères.

2. Procédé de production des composés de formule (I) suivant la revendication 1 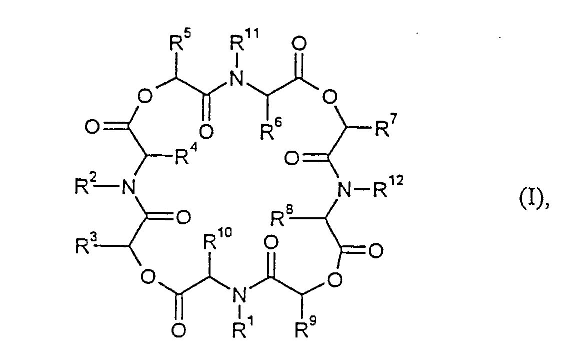 dans laquelle les restes R¹ à R¹² ont la définition indiquée dans la revendication 1,
**caractérisé en ce qu'**on cyclise des octadepsipeptides à chaîne ouverte de formule (II) 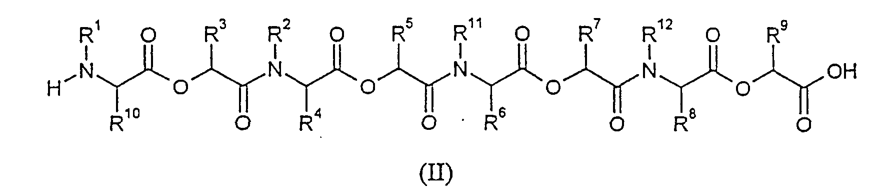 dans laquelle
R¹ à R¹² ont la définition indiquée ci-dessus
en présence d'un diluant et en présence d'un réactif de couplage.

3. Compositions endoparasiticides, **caractérisées par** une teneur en au moins un composé de formule (I) suivant la revendication 1.

4. Utilisation de composés de formule (I) suivant la revendication 1, pour la préparation de compositions endoparasiticides.
